(19) Europäisches Patentamt
European Patent Office
Office européen des brevets

(11) Veröffentlichungsnummer: **0 312 783 B1**

# (12) EUROPÄISCHE PATENTSCHRIFT

(45) Veröffentlichungstag der Patentschrift: **11.11.92**

(51) Int. Cl.$^5$: **C07D 207/28**, //A61K31/40

(21) Anmeldenummer: **88115417.3**

(22) Anmeldetag: **21.09.88**

## (54) Verfahren zur Herstellung von Erdalkalimetallsalzen der 2-Pyrrolidon-5-carbonsäure.

(30) Priorität: **17.10.87 DE 3735264**

(43) Veröffentlichungstag der Anmeldung:
**26.04.89 Patentblatt 89/17**

(45) Bekanntmachung des Hinweises auf die Patenterteilung:
**11.11.92 Patentblatt 92/46**

(84) Benannte Vertragsstaaten:
**AT BE CH DE ES FR GB GR IT LI LU NL SE**

(56) Entgegenhaltungen:

**JOURNAL OF THE AMERICAN CHEMICAL SOCIETY, Band 64, Nr. 5, 8. Mai 1942, Seiten 1017-1020; E.H. HUNTRESS et al.: "Identification of organic compounds. VI. The preparation of p-nitrobenzylpyridinium salts of aromatic sulfonic acids"**

**CHEMICAL ABSTRACTS, Band 105, Nr. 13, 29. September 1986, Seite 717, Zusammenfassung Nr. 115402m, Columbus, Ohio, US**

**CHEMICAL ABSTRACTS, Band 86, Nr. 17, 25. April 1977, Seite 587, Zusammenfassung Nr. 121776s, Columbus, Ohio, US**

**JOURNAL OF THE AMERICAN CHEMICAL SOCIETY, Band 64, Nr. 5, 8. Mai 1942, Seiten 1021-1022; N. LICHTENSTEIN: "Preparation of gamma-alkylamides of glutamic acid"**

(73) Patentinhaber: **Degussa Aktiengesellschaft**
**Weissfrauenstrasse 9**
**W-6000 Frankturt am Main 1(DE)**

(72) Erfinder: **Drauz, Karlheinz, Dr.**
**Zur Marienruhe 13**
**W-6463 Freigericht 1(DE)**
Erfinder: **Krimmer, Hans-Peter, Dr.**
**Am Weissen Turm 48**
**W-6000 Frankfurt am Main 60(DE)**
Erfinder: **Werner, Silvia**
**Goethestrasse 4**
**W-8756 Kahl(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 312 783 B1

## Beschreibung

Die Erfindung betrifft ein Verfahren zur Herstellung von Erdalkalimetallsalzen der 2-Pyrrolidon-5-carbonsäure der Formel

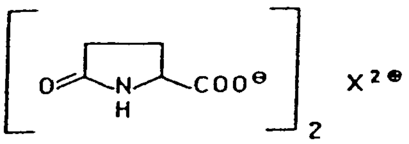

(I),

in der $X^{2+}$ ein Erdalkalimetallion bedeutet, durch Erhitzen eines einbasischen Erdalkalimetallsalzes der Glutaminsäure der Formel

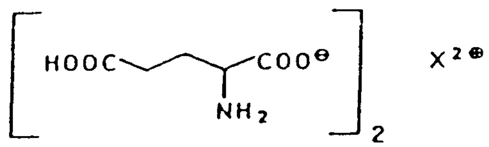

(II).

Die Erdalkalimetallsalze der 2-Pyrrolidon-5-carbonsäure sind von vielfältigem Interesse. So wird z. B. Magnesium-pyroglutamat als pharmazeutisches Präparat bei Magnesiummangelkrankheiten appliziert (FR-OS 2 546 064). Calzium-pyroglutamat hat sich als besonders effizientes Agens zur Calzium-Reinsertion in Knochensubstanz bei akutem Calziummangel bewährt [E. Moczar, B. Phan Dinh Tuy, L. Robert; Rhumatologie (Paris), 8, 71 (1979)]. Auch als Korrosionsinhibitor bei Projektil-Sprengstoffen findet Calzium-pyroglutamat Verwendung [Gor. Rep. Announce. Index (U.S.) 79, 227 (1979)].

Die Synthese der Erdalkalimetall-pyroglutamate erwies sich bisher jedoch als relativ umständlich. So wurde die Cyclisierung von einem Gemisch aus L-Glutaminsäure und Calziumoxid in wäßriger Lösung bei 140 °C und erhöhtem Druck beschrieben (ES-PS 528 010). Nachteilig ist hier die Tatsache, daß man nur eine wäßrige Lösung des Calzium-pyroglutamats erhält, sowie daß eine vollständige Racemisierung zum D,L-Produkt stattfindet, während das in den pharmazeutischen Präparationen verwendete Produkt nur das L-Enantiomere enthält, da nur dieses physiologisch wirksam ist.

Eine andere Möglichkeit der Darstellung besteht in der Umsetzung von L-Pyroglutaminsäure mit Erdalkalimetallhydroxiden, -oxiden oder -carbonaten in Wasser (FR-OS 2 546 064), jedoch entsteht hierbei auch lediglich wieder eine wäßrige Lösung des Salzes. Außerdem wird das Problem verschoben auf die Darstellung der L-Pyroglutaminsäure, die durch Erhitzen von L-Glutaminsäure in konzentrierter wäßriger Lösung nur unter starker Racemisierung und Ausbildung eines Gleichgewichts aus Ausgangsprodukt und Endprodukt gebildet wird. Ein Trennungsschritt ist zur Synthese reiner L-Pyroglutaminsäure unumgänglich (P.M. Hardy, Synthesis 1978, 290). Auch das direkte Erhitzen von L-Glutaminsäure in der Schmelze auf 180 bis 185 °C ist von Racemisierung und, speziell in diesem Fall, von sehr starker Zersetzung begleitet [N. Lichtenstein, N. Gertner, J. Am. Chem. Soc. 64, 1021 (1964)].

Schließlich wurde zur Darstellung des Magnesium-pyroglutamats die Umsetzung von Calcium-pyroglutamat mit Magnesiumsulfat beschrieben (FR-PS M 3593), die jedoch ebenfalls das Problem nur auf die Synthese des Calzium-pyroglutamats verlagert.

Aufgabe der vorliegenden Erfindung ist es, die bekannten Verfahren so zu modifizieren, daß die Reaktion auch unter Erhalt der optischen Aktivität möglich wird. Das Verfahren soll dabei mit möglichst einfachen Mitteln durchführbar sein.

Das erfindungsgemäße Verfahren ist nun dadurch gekennzeichnet, daß man das einbasische Erdalkalimetallsalz der Glutaminsäure der Formel

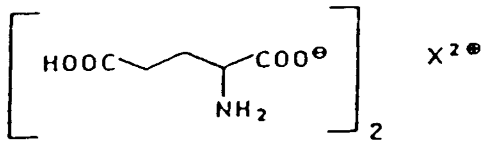

(II),

in der $X^{2+}$ wieder das entsprechende Erdalkalimetallion bedeutet, in Substanz und unter Beibehaltung des festen Zustandes auf eine Temperatur zwischen 120 °C und 250 °C erhitzt, bis das Reaktionswasser der intramolekularen Kondensation vollständig ausgetrieben ist.

"In Substanz" bedeutet hierbei in Abwesenheit irgendeines Lösungsmittels oder eines sonstigen flüssigen wärmeübertragenden Mittels.

Das Erhitzen des Erdalkalimetallsalzes der Formel (11) kann sowohl diskontinuierlich als auch kontinuierlich, z.B. in einem mit einer Einrichtung zur Zwangsförderung ausgerüsteten Reaktor, wie einem Extruder, erfolgen.

Bei der Durchführung des erfindungsgemäßen Verfahrens werden die einbasischen Erdalkalimetallsalze der Glutaminsäure, die man sehr einfach aus wohlfeiler Glutaminsäure und den entsprechenden Erdalkalihydroxiden, -oxiden oder -carbonaten erhält, in fester Form auf eine Temperatur zwischen 120 °C und 250 °C erhitzt.

Im Falle des Magnesium-glutamats hat sich z.B. eine Temperatur von 190 °C als optimal erwiesen. Es beginnt eine Abspaltung von Wasser und die Bildung des Magnesium-pyroglutamats findet spontan statt. Im Gegensatz zum Verhalten von Alkalimetallsalzen der Glutaminsäure bzw. Pyroglutaminsäure findet jedoch beim Cyclisieren kein Schmelzen statt, d.h. auch die gebildeten Erdalkalimetall-pyroglutamate sind weiterhin ein Feststoff. Es handelt sich also beim erfindungsgemäßen Verfahren um eine Feststoffreaktion. Da das gebildete Reaktionswasser die Eigenschaft hat, die feste Mischung aus Ausgangs- und Endprodukt zu verklumpen, empfiehlt es sich, diese während des Kondensationsprozesses ständig zu rühren und so neu zu vermischen. Auch das Anlegen von Vakuum ist vorteilhaft, da so das gebildete Wasser schneller aus dem Feststoffgemisch entfernt wird. Als optimales Reaktionsgefäß für kleinere bis mittelgroße Substanzmengen hat sich deshalb ein Rotationsverdampfer bewährt, dessen rotierende Blase in ein auf die optimierte Temperatur erwärmtes ölbad eintaucht und an dem ein Vakuum von 50 mbar angelegt wird. Sobald die Reaktion beendet ist, d.h. keine Wasserabspaltung mehr stattfindet, ist das Produkt wieder gut rieselfähig und kristallin und optisch nicht vom Ausgangsprodukt zu unterscheiden.

Wird die Temperatur des ölbads nicht wesentlich ( > 20 °C) über die optimierte Reaktionstemperatur erhoht, so findet keine Zersetzung und, bei Einsatz von enantiomerenreinen Ausgangsprodukten, auch keine Racemisierung statt. Die HPLC-analytisch kontrollierbare Umsetzung ist quantitativ. Auch mit Ninhydrin kann keine Glutaminsäure mehr nachgewiesen werden.

Die optimierten Reaktionstemperaturen sind für jedes Erdalkalimetallsalz leicht verschieden, sie liegen jedoch alle im Bereich zwischen 170 °C und 220 °C. Die Reaktion findet im Fall des Magnesium- und Calziumglutamats auch schon ab 120 °C statt, jedoch erhöht sich hierbei die Reaktionszeit drastisch, so daß sich die Anwendung einer höheren Temperatur als vorteilhafter erweist.

Für analytische Zwecke kann man eine verdünnte wäßrige Lösung des jeweiligen Erdalkalimetall-pyroglutamats über eine stark saure Ionenaustauschersäule (z.B. Duolite C26) entsalzen und erhält nach Entfernen des Wassers unter vermindertem Druck quantitativ die freie Pyroglutaminsäure.

Alle Erdalkalimetallsalze der Glutaminsäure, also die Beryllium-, Magnesium-, Calzium-, Strontium- und Bariumsalze, können nach dem erfindungsgemäßen Verfahren hergestellt werden.

Die Erfindung wird durch die nachfolgenden Beispiele näher erläutert:

Beispiel 1:

In einem 500 ml Rundkolben wurden 60 g (0,160 Mol) Magnesium-L-glutamat-tetrahydrat am Rotationsverdampfer bei einem Vakuum von 50 mbar in ein auf 190 °C vorgeheiztes ölbad getaucht. Die Abspaltung von Wasser und die Bildung des Magnesium-L-pyroglutamats fing nach 2 Minuten an und war nach 45 Minuten beendet, wobei es sich zu jedem Zeitpunkt der Reaktion um einen Feststoff handelte.

Es ergaben sich 43 g (99,5 % der Theorie) eines farblosen Pulvers vom Schmelzpunkt > 225 °C. Eine HPLC-Analyse [$NH_2$-Säule, 3 µm, 250 x 4,6 mm; Laufmittel: Acetonitril/0,05 M $KH_2PO_4$ (6:4; v/v), UV-Detektor 210 nm] des Produktes zeigte vollständige Umwandlung in Magnesium-L-pyroglutamat. Magnesium-L-glutamat konnte auch in Spuren nicht mehr detektiert werden ($R_t$-Differenz: Mg-L-pyroglута-

mat: Mg-L-glutamat ca. 6 Minuten).

Zur Überführung in L-Pyroglutaminsäure wurden 10 g des Produktes in 100 ml Wasser gelöst und über eine Ionenaustauschsäule [Duolite C26, $H^+$-Form, (11 cm x 2,5 cm; 30 g)] gegeben. Nach dem Eluieren und Entfernen des Wassers unter vermindertem Druck ergaben sich 9 g (quantitativ) L-Pyroglutaminsäure von Schmelzpunkt:155-156 °C [vgl. H. Gibian, E. Klieger, Liebigs Ann. Chem. 640, 145 (1961): Schmelzpunkt: 156-157 °C] . $[\alpha]_D^{25}$ : -11,8° (c = 4, $H_2O$) -[vgl. A.C. Kibrick, J. Biol. Chem., 174, 845 (1948): $[\alpha]_D^{25}$ : -11,7 ° (c = 4, $H_2O$)].

Beispiel 2:

Analog Beispiel 1 wurden 10 g (27,16 mMol) Calzium-L-glutamat-dihydrat innerhalb von 20 Minuten zwischen 150 °C und 170 °C als Feststoff cyclisiert. Es ergaben sich 8,0 g (99 % der Theorie) Calzium-L-pyroglutamat als farbloses Pulver.

Beispiel 3:

Analog Beispiel 1 wurde 1 g (2,96 mMol) Beryllium-L-glutamat-dihydrat innerhalb von 10 Minuten bei einer Temperatur von 190 °C bis 200 °C als Feststoff cyclisiert. Es ergaben sich 0,75 g (95 % der Theorie) Beryllium-L-pyroglutamat.

Beispiel 4:

Analog Beispiel 1 wurde 1 g (2,4 mMol) Strontium-L-glutamat-dihydrat innerhalb von 10 Minuten bei einer Temperatur von 190 °C bis 210 °C als Feststoff cyclisiert. Es ergaben sich 0,8 g (97 % der Theorie) Strontium-L-pyroglutamat.

Beispiel 5:

Analog Beispiel 1 wurden 5,0 g (10,7 mMol) Barium-L-glutamat-dihydrat innerhalb von 20 Minuten bei einer Temperatur von 200 °C bis 220 °C als Feststoff cyclisiert. Es ergaben sich 4,1 g (97 % der Theorie) Barium-L-pyroglutamat als farbloses Pulver.

**Patentansprüche**

**1.** Verfahren zur Herstellung von Erdalkalimetallsalzen der 2-Pyrrolidon-5-carbonsäure der Formel

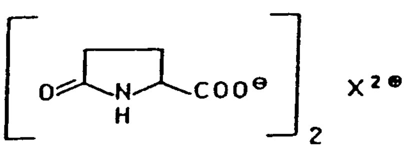

(I),

in der $X^{2+}$ ein Erdalkalimetallion bedeutet,
durch Erhitzen eines einbasischen Erdalkalimetallsalzes der Glutaminsäure der Formel

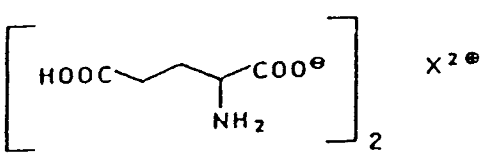

(II),

in der $X^{2+}$ wieder das entsprechende Erdalkalimetallion bedeutet,
**dadurch gekennzeichnet,**
daß man das Erdalkalisalz der Glutaminsäure in Substanz und unter Beibehaltung des festen Zustandes auf eine Temperatur zwischen 120 °C und 250 °C erhitzt, bis das Reaktionswasser der intramolekularen Kondensation vollständig ausgetrieben ist.

2. Verfahren nach Anspruch 1,
**dadurch gekennzeichnet,**
daß das Erhitzen und das Austreiben des Reaktionswassers diskontinuierlich erfolgt.

3. Verfahren nach Anspruch 1,
**dadurch gekennzeichnet,**
daß das Erhitzen und das Austreiben des Reaktionswassers kontinuierlich erfolgt.

4. Verfahren nach einem der Ansprüche 1 bis 3,
**dadurch gekennzeichnet,**
daß man das Erhitzen und das Austreiben des Reaktionswassers unter Vakuum vornimmt.

5. Verfahren nach Anspruch 4,
**dadurch gekennzeichnet,**
daß man das Reaktionswasser bei einem Druck von etwa 50 mbar austreibt.

**Claims**

1. A process for the preparation of alkaline earth metal salts of 2-pyrrolidone-5-carboxylic acid corresponding to the following formula:

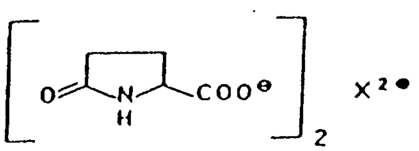

(I),

in which $X^{2+}$ denotes an alkaline earth metal ion by heating a monobasic alkaline earth metal salt of glutamic acid corresponding to the following formula:

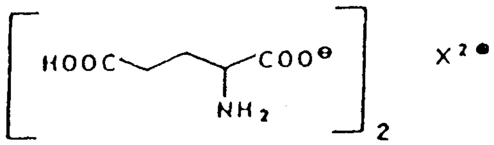

(II),

in which $X^{2+}$ again denotes the alkaline earth metal ion, characterised in that the alkaline earth metal salt of glutamic acid is heated solvent free and with maintenance of the solid state to a temperature of from 120°C to 250°C until the water of reaction from the intramolecular condensation has been completely driven off.

2. A process according to claim 1, characterised in that heating and driving off of the water of reation is carried out intermittently.

3. A process according to claim 1, characterised in that heating and the driving off of the water of reaction is carried out continuously.

4. A process according to one of the claims 1 to 3, characterised in that heating and the driving off of the water of reaction is carried out under vacuum.

5. A process according to claim 4, characterised in that the water of reaction is driven off at a pressure of about 50 mbar.

**Revendications**

1. Procédé de préparation de sels de métaux alcalino-terreux de l'acide 2-pyrrolidone-5-carboxylique de formule

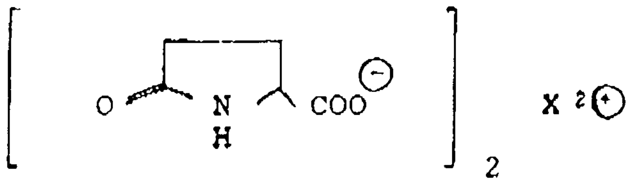

(I),

dans laquelle $X^{2+}$ représente un ion de métal alcalino-terreux, par chauffage d'un sel métallique alcalino-terreux monobasique de l'acide glutamique de formule

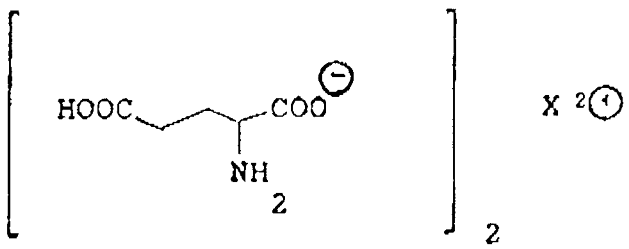

(II),

dans laquelle $X^{2+}$ représente aussi l'ion métallique alcalino-terreux correspondant, caractérisé en ce qu'on chauffe le sel alcalino-terreux de l'acide glutamique en masse en le conservant à l'état solide entre 120 °C et 250 °C, jusqu'à ce que l'eau de réaction de la condensation intramoléculaire soit complètement éliminée.

2. Procédé selon la revendication 1, caractérisé en ce que le chauffage et l'élimination de l'eau de réaction se fait de façon discontinue.

3. Procédé selon la revendication 1, caractérisé en ce que le chauffage et l'élimination de l'eau de réaction se font en continu.

4. Procédé selon l'une des revendications 1 à 3, caractérisé en ce qu'on réalise le chauffage et l'élimination de l'eau de réaction sous vide.

5. Procédé selon la revendication 4, caractérisé en ce qu'on élimine l'eau de réaction sous une pression d'environ 50 mbars.